# EUROPEAN PATENT SPECIFICATION

(11) **EP 1 826 267 B1**
(45) Date of publication and mention of the grant of the patent: **11.04.2012**
(21) Application number: 06090029.7
(22) Date of filing: 22.02.2006
(51) Int. Cl.: C12N 9/14, C12N 15/52, C07D 209/00, C07D 209/20

(54) **Novel halogenase**
Neue Halogenase
Nouvelle halogénase

(43) Date of publication of application: 29.08.2007
(73) Proprietor: B.R.A.I.N. Biotechnology Research And Information Network AG, 64673 Zwingenberg (DE)
(72) Inventor: Fischer, Carsten c/o Combinature Biopharm AG, 13125 Berlin (DE); Hornung, Andreas c/o Combinature Biopharm AG, 13125 Berlin (DE); Moeller, Annett c/o Combinature Biopharm AG, 13125 Berlin (DE); Pelzer, Stefan c/o Combinature Biopharm AG, 13125 Berlin (DE); Puetter, Vera c/o Combinature Biopharm AG, 13125 Berlin (DE); Renner, Ulf-Dietrich c/o Combinature Biopharm AG, 13125 Berlin (DE); Wohlert, Sven-Eric c/o Combinature Biopharm AG, 13125 Berlin (DE)
(74) Representative: Jungblut, Bernhard Jakob

(56) References cited:
- EP-A- 1 443 113
- WO-A-00/77182
- DATABASE UniProt [Online] 1 October 2002 (2002-10-01), "Tryptophan halogenase (Putative halogenase of tryptophan)." XP002382317 retrieved from EBI accession no. UNIPROT:Q8KHZ8 Database accession no. Q8KHZ8 -& ONAKA H ET AL: "CHARACTERIZATION OF THE BIOSYNTHETIC GENE CLUSTER OF REBECCAMYCIN FROM LECHEVALIERIA AEROCOLONIGENES ATCC 39243" BIOSCIENCE BIOTECHNOLOGY BIOCHEMISTRY, JAPAN SOC. FOR BIOSCIENCE, BIOTECHNOLOGY AND AGROCHEM, TOKYO, JP, vol. 67, no. 1, 2003, pages 127-138, XP009049256 ISSN: 0916-8451
- DATABASE UniProt [Online] 8 November 2005 (2005-11-08), "Tryptophan halogenase." XP002382319 retrieved from EBI accession no. UNIPROT:Q3F6K5 Database accession no. Q3F6K5
- DATABASE Geneseq [Online] 5 September 2001 (2001-09-05), "Pseudomonas fluorescens D-tryptophan halogenase PrnA." XP002382320 retrieved from EBI accession no. GSP:AAB82506 Database accession no. AAB82506 -& WO 01/44447 A (SYNGENTA PARTICIPATIONS AG; STEFFENS, JOHN; BATIE, CHRIS; DIETZ, JON,) 21 June 2001 (2001-06-21)
- DATABASE UniProt [Online] 1 May 1997 (1997-05-01), "Tryptophan halogenase PrnA." XP002382321 retrieved from EBI accession no. UNIPROT:P95480 Database accession no. P95480 -& HAMMER PHILIP E ET AL: "Four genes from Pseudomonas fluorescens that encode the biosynthesis of pyrrolnitrin" APPLIED AND ENVIRONMENTAL MICROBIOLOGY, WASHINGTON,DC, US, vol. 63, no. 61, 1997, pages 2147-2154, XP002146269 ISSN: 0099-2240
- DATABASE UniProt [Online] 1 May 2000 (2000-05-01), "Tryptophan halogenase." XP002382322 retrieved from EBI accession no. UNIPROT:Q9RPG8 Database accession no. Q9RPG8 -& HAMMER PHILIP E ET AL: "Conservation of the pyrrolnitrin biosynthetic gene cluster among six pyrrolnitrin-producing strains" FEMS MICROBIOLOGY LETTERS, vol. 180, no. 1, 1 November 1999 (1999-11-01), pages 39-44, XP002382310 ISSN: 0378-1097

### SEQUENCE LISTING

<110> Combinature Biopharm AG
<120> Novel Halogenases
<130> COM/EP/0513
<160> 30
<170> PatentIn version 3.2
<210> 1
   <211> 540
   <212> PRT
   <213> Streptomyces Tü1892
<400> 1
   Val Ser Asp Asn Ile Glu His Ile Val Ile Leu Gly Gly Gly Thr Ala
   1               5                   10                  15
   Gly Trp Met Ser Ala Ala Tyr Leu Gly Lys Ala Leu Gln Gly Ala Val
               20                  25                  30
   Arg Ile Thr Val Leu Glu Ala Pro Ala Ile Pro Lys Ile Gly Val Gly
           35                  40                  45
   Glu Ala Thr Val Pro Asn Leu Gln Arg Val Phe Phe Asp Phe Leu Gly
       50                  55                  60
   Ile Pro Glu Asp Glu Trp Met Arg Glu Cys Asn Ala Ser Phe Lys Met
   65                  70                  75                  80
   Ala Val Lys Phe Ile Asn Trp Arg Thr Pro Gly Glu Gly Ser Pro Thr
                   85                  90                  95
   Ala Arg Thr Leu Asn Gly Arg Pro Asp His Phe Tyr His Pro Phe Gly
               100                 105                 110
   Ile Leu Pro Gln Glu Asp Gly Val Pro Ile Thr His His Trp Ser Asp
           115                 120                 125
   Arg Ala Ala Arg Gly Glu Thr Asp Leu Pro Phe Asp Tyr Ser Cys Phe
       130                 135                 140
   Val Asp Pro Val Leu Met Asp Arg Asn Leu Ala Pro Arg His Pro Asp
   145                 150                 155                 160
   Gly Arg Pro Ala Thr Arg Tyr Ala Trp His Phe Asp Ala His Leu Val
                   165                 170                 175
   Ala Asp Tyr Leu Arg Arg Trp Ser Val Glu Lys Gln Gln Val Gln His
               180                 185                 190
   Val Gln Asp Arg Met Val Asp Val Gln Leu Asp Gln Asn Gly Phe Ile
           195                 200                 205
   Arg Ser Leu Arg Thr Glu Ser Gly Arg Thr Ile Glu Gly Asp Leu Phe
       210                 215                 220
   Ile Asp Cys Ser Gly Phe Arg Gly Ala Leu Ile Asn Gln Val Met Glu
   225                 230                 235                 240
   Glu Pro Phe Leu Asp Gln Ser Asp His Leu Leu Cys Asp Ser Ala Leu
                   245                 250                 255
   Ala Cys Pro Val Pro His Asp Asp Ala Ala Arg Gly Ile Glu Pro Tyr
               260                 265                 270
   Thr Ser Ser Ile Ala Met Lys Ser Gly Trp Ala Trp Lys Thr Pro Leu
           275                 280                 285
   Leu Gly Arg Phe Gly Thr Gly Tyr Val Tyr Ser Ser Ser Phe Ala Thr
       290                 295                 300
   Glu Asp Glu Ala Val Arg Asp Phe Thr Ala Leu Trp Gly Ile Ser Ala
   305                 310                 315                 320
   Asp Arg Glu Phe Asn Arg Ile Arg Phe Arg Val Gly Arg Asn Arg Arg
                   325                 330                 335
   Ser Trp Val Lys Asn Cys Val Ser Ile Gly Leu Ser Ser Cys Phe Leu
               340                 345                 350
   Glu Pro Leu Glu Ser Thr Gly Leu Tyr Phe Thr Tyr Ala Ala Ile Tyr
           355                 360                 365
   Gln Leu Ala Lys His Phe Pro Asp Lys Ser Phe Ser Pro Ile Phe Ala
       370                 375                 380
   Asp Arg Phe Asn Lys Glu Ile Ala Ala Met Tyr Asp Glu Ser Arg Asp
   385                 390                 395                 400
   Phe Leu Gln Ala His Phe Tyr Phe Ser Pro Arg Glu Asp Thr Asp Phe
                   405                 410                 415
   Trp Arg Ala Asn Lys Asp Leu Thr Leu Gly Glu Glu Ile Arg Glu Lys
               420                 425                 430
   Ala Glu Met Tyr Leu Lys Gly Ile Pro Val Asn Leu Pro Thr Ala Asp
                   435                 440                 445
   Glu Ser Thr Tyr Tyr Gly Asn Phe Glu Ala Glu Phe Arg Asn Phe Trp
       450                 455                 460
   Thr Asn Gly Ser Tyr Tyr Cys Ile Phe Ala Gly Leu Gly Met Leu Pro
   465                 470                 475                     480
   Glu Arg Pro Ile Ala Pro Leu Ala Tyr Gly Arg Asp Thr Arg Ala Ser
                   485                 490                 495
   Ala Arg Arg Met Phe Asp Gly Val Ala Arg Arg Gln Arg Glu Leu Ser
               500                 505                 510
   Gln Thr Leu Pro Thr Met Tyr Asp Tyr Leu Arg Asp Phe His Gly Ala
           515                 520                 525
   Ser Ala Gly Ser Gly Asp Gly Asp Arg His Ala Arg
       530                 535                 540
<210> 2
   <211> 531
   <212> PRT
   <213> Streptomyces Tü 4042
<400> 2
   Met Ala Ala Asp Asn Arg Ile Arg Lys Ile Val Ile Leu Gly Gly Gly
   1               5                   10                  15
   Thr Ala Gly Trp Met Thr Ala Ala Tyr Leu Ser Lys Ala Leu Gly Ser
               20                  25                  30
   Ser Val Gly Ile Thr Val Leu Glu Ala Pro Thr Val Pro Arg Ile Gly
           35                  40                  45
   Val Gly Glu Ala Thr Ile Pro Asn Leu Gln Lys Val Phe Phe Asp Phe
       50                  55                  60
   Leu Gly Leu Pro Glu Glu Glu Trp Met Arg Glu Cys Asn Ala Ser Phe
   65                  70                  75                  80
   Lys Met Gly Ile Arg Phe Ile Asn Trp Arg Thr Ala Gly Ala Gly His
                   85                  90                  95
   Ser Val Ala Arg Pro Trp Gly Arg Arg Leu Asp His Phe Asp His Ser
               100                 105                 110
   Phe Gly Leu Leu Pro Ser His Asp Asn Val Pro Leu Ser His Tyr Trp
           115                 120                 125
   Thr Arg Lys Lys Leu Asn Asn Ser Thr Glu Glu Ser Phe Asp Arg Ala
       130                 135                 140
   Cys Tyr Ser Gln Pro Ala Ile Phe Asp Lys Lys Leu Ser Pro Arg Phe
   145                 150                 155                 160
   Met Asp Gly Thr Arg Trp Thr Ser Tyr Ala Trp His Phe Asp Ala Gln
                   165                 170                 175
   Leu Val Ala Asp Phe Leu Arg Arg Phe Ser Thr Glu Lys Gln Gly Val
               180                 185                 190
   Val His Val Glu Asp Lys Tyr Val His Ala Glu Leu Asp Glu Gln Gly
           195                 200                 205
   His Val Ala Ala Ile Thr Thr Glu Thr Gly Val Arg Leu Asp Gly Asp
       210                 215                 220
   Leu Phe Val Asp Cys Ser Gly Phe Arg Ser Leu Leu Met Asn Gln Val
   225                 230                 235                 240
   Met Gln Glu Pro Phe Leu Asp Met Ser Asp His Leu Leu Asn Asp Arg
                   245                 250                 255
   Ala Val Ala Thr Gln Ile Pro His Asp Asp Arg Thr Asp Gly Val Glu
               260                 265                 270
   Pro Phe Thr Ser Ser Ile Ala Met Ser Ser Gly Trp Thr Trp Lys Ile
           275                 280                 285
   Pro Met Leu Gly Arg Phe Gly Thr Gly Tyr Val Tyr Ser Ser Arg Phe
       290                 295                 300
   Ala Ser Gln Asp Glu Ala Thr Leu Glu Phe Cys Arg Met Trp Gly Ile
   305                 310                 315                 320
   Asp Pro Glu Arg Gln Pro Leu Asn His Ile Lys Phe Arg Val Gly Arg
                   325                 330                 335
   Asn Arg Arg Ala Trp Val Lys Asn Val Val Gly Ile Gly Leu Ser Ser
               340                 345                 350
   Cys Phe Leu Glu Pro Leu Glu Ser Thr Gly Ile Tyr Phe Thr Tyr Ala
           355                 360                 365
   Ala Ile Tyr Gln Leu Val Lys His Phe Pro Asp Lys Arg Phe Asp Pro
       370                 375                 380
   Val Leu Thr Gln Arg Phe Asn Leu Glu Ile Glu Arg Met Phe Asp Asp
   385 390 395 400
   Thr Arg Asp Phe Ile Gln Gly His Phe Ser Phe Ala Pro Arg Asn Asp
   405 410 415
   Thr Pro Phe Trp Arg Ala Cys Lys Glu Leu Glu Leu Thr Glu Ser Phe
   420 425 430
   Gln Glu Lys Val Ala Phe Tyr Lys Ser Gly Leu Gly Val Asn Leu Pro
   435 440 445
   Ile Ser Asp Glu Asn Asp Tyr Tyr Ser Asn Phe Glu Met Glu Phe Glu
   450 455 460
   Asn Phe Trp Ser Asn Ala Asn Tyr Tyr Cys Val Phe Thr Gly Leu Glu
   465 470 475 480
   Leu Phe Pro Glu Arg Ser Leu Pro Leu Leu Asp Leu Arg Pro Glu Ser
   485 490 495
   Val Asp Gly Ala Glu Ala Ala Phe His Arg Leu Lys Glu Arg Thr Asp
   500 505 510
   Gln Leu Ser Asn Thr Leu Pro Thr Leu Tyr Asp Tyr Leu Arg His Leu
   515 520 525
   His Ser Lys
   530
<210> 3
   <211> 537
   <212> PRT
   <213> Streptomyces nodosus
<400> 3
   Met Thr Gly Thr Ser Thr Ala Thr Asp Asn Ala Ile Lys Lys Val Val
   1 5 10 15
   Ile Leu Gly Gly Gly Thr Ala Gly Trp Met Thr Ala Ala Tyr Leu Gly
   20 25 30
   Lys Ala Leu Gln Ser Thr Val Asp Val Thr Val Leu Glu Ala Pro Ser
   35 40 45
   Ile Pro Arg Ile Gly Val Gly Glu Ala Thr Val Pro Asn Leu His Arg
   50 55 60
   Val Leu Phe Asp Phe Leu Gly Ile Glu Glu Glu Glu Trp Met Arg Glu
   65 70 75 80
   Cys Asn Ala Ser Tyr Lys Met Ala Val Arg Phe Ile Asn Trp Arg Thr
   85 90 95
   Pro Gly Lys Gly Gln Ser Ser Pro Arg Glu Leu Asp Lys Gly Gly Pro
   100 105 110
   Asp His Phe Tyr His Pro Phe Gly Ile Ser Pro Glu His Asp Asn Thr
   115 120 125
   Pro Met Ser His Tyr Trp Phe Lys Asn Lys Tyr Glu Gly Lys Thr Asp
   130 135 140
   Glu Pro Tyr Asp Tyr Ala Cys Phe Arg Glu Ala Pro Leu Met Asp Ala
   145 150 155 160
   Asn Lys Ala Pro Arg His Leu Asp Gly Thr Ser Pro Thr Arg Tyr Ala
   165 170 175
   Trp His Phe Asp Ala Ala Leu Val Ala Asp Phe Leu Arg Arg Phe Ala
   180 185 190
   Thr Gly Lys Gln Gly Val His His Ile Gln Asp Glu Met Thr Arg Val
   195 200 205
   Glu Lys Asp Glu Arg Gly Phe Val Lys Ala Leu His Thr Lys Glu Gly
   210 215 220
   Arg Val Leu Asp Ala Asp Leu Phe Val Asp Cys Ser Gly Phe Arg Ser
   225 230 235 240
   Leu Leu Met Asn Gln Ala Met Glu Glu Pro Phe Val Asp Met Ser Asp
   245 250 255
   Gln Leu Leu Cys Asp Arg Ala Val Ala Thr Ala Val Pro His Asp Asp
   260 265 270
   Glu Ala Asn Gly Val Glu Pro Tyr Thr Ser Ala Ile Ala Met Ser Ser
   275 280 285
   Gly Trp Thr Trp Lys Ile Pro Met Leu Gly Arg Phe Gly Thr Gly Tyr
   290 295 300
   Val Tyr Ser Ser Arg Phe Thr Thr Gln Glu Glu Ala Thr Ala Glu Phe
   305 310 315 320
   Cys Glu Leu Trp Gly Leu Asp Pro Glu Thr Thr Gln Phe Asn His Val
                   325                 330                 335
   Lys Phe Arg Val Gly Arg Asn Arg Arg Ala Trp Val Lys Asn Val Ile
               340                 345                 350
   Gly Ile Gly Leu Ser Ser Ala Phe Leu Glu Pro Leu Glu Ser Thr Gly
           355                 360                 365
   Ile Tyr Phe Ile Thr Ala Ala Ala Tyr Gln Leu Ala Lys His Phe Pro
       370                 375                 380
   Asp Lys Thr Phe Asn Gln Ala Leu Val Asp Asn Phe Asn Lys Glu Ile
   385                 390                 395                 400
   Glu Val Met Phe Asp Asp Thr Arg Asp Phe Ile Gln Thr His Phe Tyr
                   405                 410                 415
   Phe Ala Pro Arg Thr Asp Ser Pro Phe Trp Arg Ala Asn Lys Glu Leu
               420                 425                 430
   Lys Leu Ala Pro Ser Ile Arg Glu Lys Ile Asp Ala Tyr Lys Ala Gly
           435                 440                 445
   Leu Pro Val Asn Pro Ala Ala Thr Asp Asp Thr Thr Tyr Tyr Gly Asn
       450                 455                 460
   Phe Glu Ala Glu Phe Arg Asn Phe Trp Thr Asn Glu Arg Tyr Tyr Cys
   465                 470                 475                 480
   Ile Phe Ala Gly Leu Gly Leu Glu Pro Asp Ala Pro Leu Pro Ile Leu
                   485                 490                 495
   Tyr His Lys Pro Glu Ser Val Ala Ala Ala Gln Ala Met Phe Thr Lys
               500                 505                 510
   Val Lys Lys Glu Gln Gln Glu Leu Leu Asn Ser Leu Pro Ser Thr Tyr
           515                 520                 525
   Glu Ala Leu Arg Gln Leu His Gly Lys
       530                 535
<210> 4
   <211> 527
   <212> PRT
   <213> Streptomyces Tü 4053
<400> 4
   Val Ala Arg Pro Val Gln Lys Ile Val Ile Val Gly Gly Gly Thr Ala
   1               5                   10                  15
   Gly Trp Leu Ser Ala Ala Tyr Leu Asn Arg Ala Phe Gly Ser Lys Val
               20                  25                  30
   Glu Ile Thr Leu Val Glu Ser Ala Arg Ile Gly Arg Ile Gly Val Gly
           35                  40                  45
   Glu Ala Thr Val Pro Thr Leu Arg Thr Thr Leu Ala Phe Leu Gly Met
       50                  55                  60
   Lys Glu Glu Asp Trp Met Pro Arg Thr Asn Ala Ser Phe Lys Ser Ala
   65                  70                  75                  80
   Val Arg Phe Asn Asp Trp Arg Thr Pro Arg Pro Gly Thr Asp Arg His
                   85                  90                  95
   Thr Phe Tyr His Pro Phe Phe Thr Val Pro Glu Pro Pro Val Pro Ser
               100                 105                 110
   Tyr Glu Arg Pro Phe His Lys Arg Phe Gly Arg Gly Val Ser Leu Ala
           115                 120                 125
   His Phe Trp Thr Lys Leu Arg Leu Ala Gly Asp Ser Arg Val Arg Glu
       130                 135                 140
   Thr Phe Gly Asp Ala Gly Met Ala Leu Gln His Leu Cys Glu Leu Asn
   145                 150                 155                 160
   Lys Ala Pro Lys Pro Leu Pro Gly Ser Gly Ala Pro Asp Pro Gly Phe
                   165                 170                 175
   Arg Tyr Ala Tyr His Phe Asp Ala Ala Leu Met Ala Glu Tyr Leu Arg
               180                 185                 190
   Asp Leu Ala Thr Gly Arg Gly Val Arg His Val Val Ala Asp Val Thr
           195                 200                 205
   Gln Val Thr Arg Asp Gly Arg Gly His Ile Asp Lys Val Ile Thr Glu
       210                 215                 220
   Thr Gly Ala Glu Leu Thr Gly Asp Leu Phe Leu Asp Cys Ser Gly Phe
   225                 230                 235                 240
   Arg Gly Leu Leu Ile Asn Gln Thr Leu Glu Glu Pro Phe Val Ser Ala
                   245                 250                 255
   Gln Asp Trp Leu Leu Cys Asp Ser Ala Ile Ala Leu Pro Ala Asp His
   260 265 270
   His Pro Asp Gly Ile Arg Pro Tyr Thr Ser Ala Asn Ala Arg Pro Asn
   275 280 285
   Gly Trp Ile Trp Glu Ile Pro Leu Tyr His Arg Glu Gly Thr Gly Tyr
   290 295 300
   Val Tyr Ser Ser Arg Thr Thr Thr Pro Asp Gln Ala Glu Gln Asp Leu
   305 310 315 320
   Arg Asp Phe Leu Gly Pro Arg Ala His Asp Val Pro Gly Asn His Ile
   325 330 335
   Arg Met Arg Val Gly His Asn Arg Arg Ser Trp Val Asp Asn Cys Val
   340 345 350
   Ala Ile Gly Leu Ser Ser Cys Phe Val Glu Pro Leu Glu Ser Thr Thr
   355 360 365
   Ile Ala Leu Ile Glu Tyr Gln Leu Ala Leu Leu Val Leu His Phe Pro
   370 375 380
   Asp Ser Glu Leu Asp Glu Arg Arg Gln Ala Arg Tyr Asn Glu Leu Met
   385 390 395 400
   Val Ser Ala Phe Glu Asp Leu Arg Asp Phe Ile Val Met His Tyr Ala
   405 410 415
   Leu Thr Asp Arg Thr Asp Thr Glu Phe Trp Arg Ala Val Arg Glu Asn
   420 425 430
   Pro Ile Pro Asp Ser Leu Ala Glu Lys Leu Thr Glu Tyr Ala Glu Ser
   435 440 445
   Val Ile Ile Pro Asp Gly Ser Gln Leu Arg Leu Phe Glu Thr Arg Ser
   450 455 460
   Leu Trp Ala Ile Leu Ser Gly Met Asp Phe Ala Phe Thr Lys Ala Pro
   465 470 475 480
   Pro Ser Val Glu Leu Met Ser Asp Glu Ala Ala Trp Gln Ala Phe Glu
   485 490 495
   Gln Ile Asp Lys Glu Arg Glu Ile Tyr Ala Ala Ala Leu Pro Asp Asn
   500 505 510
   Arg Thr Tyr Leu Glu Ala Val His Ala Gly Arg Ala Asp Ser Ala
   515 520 525
<210> 5
   <211> 533
   <212> PRT
   <213> Streptomyces Tü 3120
<400> 5 <210> 6
   <211> 530
   <212> PRT
   <213> Saccharothrix espanaensis
<400> 6
<210> 7
   <211> 530
   <212> PRT
   <213> Lechevalieria aerocolonigenes
<400> 7
   Met Glu Asp His Arg Ile Ser Asn Val Val Val Leu Gly Gly Gly Thr
   1               5                   10                  15
   Ala Gly Trp Met Thr Ala Ser Tyr Leu Gly Lys Ala Leu Gly Arg His
               20                  25                  30
   Ala Thr Ile Thr Val Leu Glu Ala Pro Ser Ile Pro Lys Ile Gly Val
           35                  40                  45
   Gly Glu Ala Thr Val Pro Asn Leu His Lys Thr Phe Phe Asp Phe Leu
       50                  55                  60
   Gly Ile Pro Glu Glu Glu Trp Met Arg Glu Cys Asn Ala Ser Phe Lys
   65                  70                  75                  80
   Met Ala Val Lys Phe Val Asn Trp Arg Thr Pro Gly Glu Gly Glu Ala
                   85                  90                  95
   Val Gly Arg Gln Leu Arg Gly Arg Pro Asp His Tyr Tyr His Leu Phe
   100 105 110
   Gly Leu Leu Pro Thr His Gln Asn Leu Pro Leu Ser His Tyr Trp Ala
   115 120 125
   Ala Lys Ser Leu Gly Gly Gln Thr Thr Glu Gln Phe Asp Tyr Ala Cys
   130 135 140
   Tyr Arg Glu Ser Pro Ile Ile Asp Ala Arg Leu Ser Pro Arg His Ala
   145 150 155 160
   Asp Gly Thr Lys Trp Thr Asn Tyr Ala Trp His Phe Asp Ala His Leu
   165 170 175
   Val Ala Asp Phe Leu Lys Arg Phe Ala Ile Gln Lys Gln Gly Val Val
   180 185 190
   His Val Gln Asp Glu Met Thr Asp Ala Thr Ile Asp Gln Arg Gly His
   195 200 205
   Ile Thr Ala Leu Arg Thr Lys Glu Gly Arg Thr Ile Glu Gly Asp Leu
   210 215 220
   Phe Val Asp Cys Ser Gly Phe Arg Gly Leu Leu Ile Asn Lys Leu Met
   225 230 235 240
   Arg Glu Pro Phe Leu Asp Met Ser Asp His Leu Leu Asn Asp Ser Ala
   245 250 255
   Val Ala Thr Ala Val Pro His Asp Asp Glu Ala His Gly Val Glu Pro
   260 265 270
   Ala Thr Ser Ala Ile Ala Met Lys Ser Gly Trp Thr Trp Lys Ile Pro
   275 280 285
   Met Leu Asp Arg Phe Gly Thr Gly Tyr Val Tyr Ser Ser Arg Phe Ala
   290 295 300
   Asp Ser Asp Thr Ala Ala Arg Asp Leu Cys Ala Leu Trp Asn Leu Asp
   305 310 315 320
   Pro Glu Glu Gln Pro Leu Asn His Val Lys Phe Arg Val Gly Arg Asn
   325 330 335
   Arg Arg Ala Trp Val Arg Asn Cys Val Ser Ile Gly Leu Ser Ser Cys
   340 345 350
   Phe Leu Glu Pro Leu Glu Ser Thr Gly Ile Tyr Phe Ile Thr Ala Ala
           355                 360                 365
   Leu Tyr Gln Leu Gly Lys His Phe Pro Asp Lys Arg Phe Asp Pro Val
       370                 375                 380
   Leu Ile Glu Ser Phe Asn Arg Glu Ile Glu Thr Met Phe Asp Asp Ser
   385                 390                 395                 400
   Arg Asp Phe Leu Gln Ala His Phe Thr Leu Ala Pro Arg Thr Asp Thr
                   405                 410                 415
   Pro Phe Trp Arg Ala Cys Lys Asp Leu Val Leu Pro Glu Asp Ile Gln
               420                 425                 430
   His Lys Ile Ala Gln Tyr Arg Ala Gly Leu Ala Ile Asn Met Pro Val
           435                 440                 445
   Thr Asp Glu Ala Thr Tyr Tyr Glu Asn Phe Glu Ala Glu Phe Arg Asn
       450                 455                 460
   Phe Trp Thr Asn Ser Ser Tyr Tyr Cys Ile Phe Ala Gly Leu Asp Val
   465                 470                 475                 480
   Leu Pro Asp His Pro Leu Pro Thr Leu Ala Phe Arg Pro Glu Ala Met
                   485                 490                 495
   Glu Gly Ala Glu Pro Val Phe Ala Gln Ile Lys Gln Lys Gln Ala Glu
               500                 505                 510
   Leu Leu Lys Thr Leu Pro Thr Val His Glu Tyr Leu Arg Glu Leu His
           515                 520                 525
   Gly Arg
       530
<210> 8
   <211> 1623
   <212> DNA
   <213> Streptomyces Tü 1892
<400> 8
   gtgtcggaca acatcgaaca catagtgatc ctcggcggcg gcacggcggg gtggatgagc      60
   gccgcgtatc tcggcaaggc gttacaggga gcggtgcgca tcacggttct cgaagcgcct     120
   gccattccga aaatcggggt cggcgaggcc accgtgccga acctgcagcg cgtcttcttc     180
   gatttcctcg gcatcccgga ggacgagtgg atgcgcgagt gcaacgcctc cttcaagatg     240
   gccgtgaaat tcatcaactg gcggaccccc ggtgagggaa gtcccaccgc gcgcacgctg     300
   aacggccggc cggaccactt ctaccacccg ttcggcatcc ttccgcagga ggacggcgtc     360
   ccgatcaccc accactggtc cgaccgcgcg gcgcgcggcg agaccgacct gcccttcgac     420
   tacagctgct tcgtcgaccc ggtgctgatg gaccgcaacc tcgccccgcg ccaccccgac     480
   ggccggcccg ccacccggta cgcctggcac ttcgacgccc atctggtcgc cgactacctg     540
   cgccgctggt cggtggagaa gcagcaggta cagcacgtcc aggaccggat ggtggacgtc     600
   cagctggacc agaacggctt catccgctcc ctgcgcacgg agagcgggcg caccatcgag     660
   ggcgacctgt tcatcgactg ctccggcttc cgcggcgcac tgatcaacca ggtcatggag     720
   gagccgttcc tcgaccagag cgaccacctg ctctgcgaca gcgccctggc ctgcccggtg     780
   ccgcacgacg acgcggcccg cgggatcgag ccgtacacct cgtccatcgc catgaagtcg     840
   gggtgggcct ggaagacgcc gctgctcggc cgcttcggca ccggatacgt ctattccagc     900
   tcgttcgcga cggaggacga ggcggtacgg gacttcaccg cgctctgggg gatctccgcc     960
   gaccgtgagt tcaaccggat ccggttccgg gtgggtcgta accggcggtc ctgggtgaag    1020
   aactgcgtca gcatcggtct gtcgtcgtgt tttctggagc cgctggagtc gaccggcctc    1080
   tacttcacct acgcggccat ctaccagttg gccaagcatt tcccggacaa gagcttcagt    1140
   ccgatattcg cggatcgctt caacaaggag atcgcggcga tgtacgacga gtcgcgtgat    1200
   ttcctgcagg cgcatttcta tttctcgccg cgcgaggaca cggatttctg gcgggcgaac    1260
   aaggacctga ccctcggcga ggagatccgc gagaaggcgg agatgtacct caagggcatt    1320
   cctgtcaacc tgccgaccgc cgacgagtcg acgtactacg gcaacttcga ggcggagttc    1380
   cgcaacttct ggaccaacgg cagctactac tgcatcttcg ccggcctcgg catgctgccg    1440
   gagcggccca tcgctccgct cgcgtacggc cgtgacacgc gcgcgtccgc gcggagaatg    1500
   ttcgacggtg tggcccggcg gcagcgggag ctgtcgcaga ccctgccgac catgtacgac    1560
   tatctgcgtg acttccacgg agcgtccgcc ggctccggcg acggcgaccg ccatgcccgc    1620
   tga                                                                  1623
<210> 9
   <211> 1596
   <212> DNA
   <213> Streptomyces Tü 4042
<400> 9 <210> 109
   <211> 1614
   <212> DNA
   <213> Streptomyces Nodosus
<400> 10
<210> 11
   <211> 1584
   <212> DNA
   <213> Streptomyces Tü 4053
<400> 11
   gtggcacgtc cagtccagaa gatcgtgata gtcggcggcg ggacggccgg ctggctctcc     60
   gcggcctacc tcaatcgcgc gttcggcagc aaggtcgaga tcaccctcgt ggagtcggcg    120
   cgcatcggcc ggatcggggt cggtgaggcc accgtgccga ccctgcgcac cacactggcc    180
   ttcctcggca tgaaggaaga ggactggatg ccgcggacga acgcgtcgtt caagtccgcg    240
   gtccggttca acgactggcg cacgccccgc cccggcaccg accggcacac cttctaccac    300
   ccgttcttca ccgtgcccga gccgccggtg cccagctacg agcggccgtt ccacaagcgg    360
   ttcgggcggg gtgtgtccct ggcccacttc tggaccaagc tgcggctggc cggcgactcc    420
   cgggtgcggg agaccttcgg tgacgcgggc atggcgctcc agcacctgtg cgagctgaac    480
   aaggccccga agccgctgcc cggatcgggc gccccggacc ccgggttccg ctacgcctac    540
   cacttcgacg ccgctctgat ggccgagtac ctccgggacc tggccaccgg gcgcggcgta    600
   cggcacgtgg tcgccgatgt cacgcaggtc acccgcgacg ggcgcggcca catcgacaag    660
   gtgatcaccg aaaccggcgc cgagctcacc ggcgacctgt tcctggactg ctccggcttc    720
   cgcggcctgc tgatcaacca gaccctggag gagccgttcg tcagcgcgca ggactggctg    780
   ctgtgcgact ccgccatcgc gctccccgcc gatcaccacc ccgacggcat ccgcccgtac    840
   accagcgcca acgcccgtcc gaacggctgg atctgggaga tcccgctgta ccaccgcgag    900
   ggcaccggtt acgtgtactc gagccggacg acgactccgg accaggccga gcaggacctc    960
   cgcgacttcc tgggcccgcg cgcccacgac gtaccgggca accacatccg gatgcgggtg   1020
   ggccacaacc ggcgctcgtg ggtcgacaac tgcgtggcga tcgggctgtc gagctgtttc   1080
   gtggagccgc tggagtccac caccatcgcc ctcatcgaat accagctggc actgctggtc   1140
   ctgcatttcc cggattcgga gctcgacgag cgccggcagg cccgctacaa cgagctgatg   1200
   gtgagcgcgt tcgaggatct gcgcgacttc atcgtcatgc actacgccct caccgaccgc   1260
   acggacaccg aattctggcg cgcggtacgg gagaacccga tccccgacag cctcgcggag   1320
   aaactcaccg agtacgcgga aagcgtcatc attcccgacg gcagccagct ccggctcttc   1380
   gagacgcgga gcctgtgggc gatcctttcg ggaatggact tcgcattcac caaggcgccg   1440
   ccttcggtgg agctgatgag cgacgaagcc gcctggcagg cattcgagca gatcgacaag   1500
   gagcgcgaga tatacgccgc cgcgctgccc gacaaccgca cctacctgga ggccgtccac   1560
   gccggccggg ccgactcggc gtag                                          1584
<210> 12
   <211> 1602
   <212> DNA
   <213> Streptomyces Tü 3120
<400> 12
   atgaccagca ccgacacccg gcttcagaag gtcgtcatcc tgggaggcgg cacggccggc      60
   tggatgacgg ccgcgtatct cggcaaggcg ctccagggaa ccgtcgacat caccgtcctg     120
   gaggcgccga cgatcccgcg catcggggtg ggtgaggcca ccgtccccaa tctgcagcgc     180
   tccttcttcg actacctggg gatcgccgag gacgagtgga tgcgggagtg caacgccagc     240
   ttcaagatgg cggtgcgctt cgtgaactgg cggaccggcg ggcagggcga gtcgaccgcg     300
   cgggagctgc cgggcggcgg cccggaccac ttctaccacc ccttcgggct gcttccggac     360
   tacgaccaga cgccgctgtc gcactactgg ttcaagcgca agtacgaggg ccgcaccacc     420
   gagccgttcg actacgcctg cttccgcgag cctcccgtga tggacgcgat gaaggccccg     480
   cgctggctcg acggccgtcc ggccacccgg tacgcctggc acttcgacgc ccagctggtg     540
   gccgacttcc tgcgccgctt cgccaccgag aagcagggcg tgaaccacgt ccaggacgag     600
   atggtccgtg tcgagcagga ctcccgcggg tatgtcacga cgctgcacac caagggcggg     660
   ctggccctgg acgccgacct gttcgtggac tgctccggct tccgcgggct gctgatcaac     720
   caggcggtgc aggagccctt catcgacatg agcgaccacc tgctgtgcga cagcgcggtg     780
   gcgacggccg tcccgcacga cgacgaggcg aacggggtgg agccgtacac ctcggcgatc     840
   gccatgtcct ccggctgggc ctggaagatc cccatgctgg gccgcttcgg caccggctac     900
   gtgtactcca gcaagttcac cgaccaggac accgccaccc gcgagttcgc ggacatgtgg     960
   ggcctggacg tggaggagac gaagttcaac cacatccgct tccgggtcgg ccgcaaccgc    1020
   cgcgcgtggg tgaagaacgt ggtgagcatc ggcctgtcgt cctgcttcct ggagccgctg    1080
   gagtcgaccg gcatctactt catcacggcc gcgatctacc agctggcgaa gcacttcccg    1140
   gacaagacct tcaacgaggg cctgatcgac agcttcaacc acgagatcga ggtgatgttc    1200
   gacgacaccc gcgacttcat ccaggcgcac ttcttctacg cgccgcgcaa cgacacgccg    1260
   ttctggcagg ccaacaagaa gctggtcctc cccgacaaca tcaagcagaa gatctccgcc    1320
   tacaaggccg gcctgcccat caactccccg atcaccgacg agtcgacgta ctacggcaac    1380
   ttcgaggcgg agttccgcaa cttctggacc aacggcagct actactgcat cttcgccggc    1440
   ctcggtctgg agccggacgc cccgctgccc accctcgccc accggcccga gtccgtggcc    1500
   ggcgccgaac cgctcttcga caccgtcaag cgccagcagc agaacctgct ggaaaccctc    1560
   cccagcgcct acgactacct gcgccacctg cacggcgcgt aa                       1602
<210> 13
   <211> 1593
   <212> DNA
   <213> Saccharothrix aspanaensis
<400> 13
   atggaagatc gcaggatcaa gaaagtcgtc gtgctcggtg gcggcaccgc gggctggatg 60
   gccgcggcgt acctgggcaa ggcgctgggt ccgggcgtga acatcaccgt gctggaggcg 120
   ccggcgatcc ccaagatcgg cgtcggcgag gccaccgtgc cgaacctgca caagaccttc 180
   ttcgacttcc tcggcatcgc cgaggaggac tggatgcggg agtgcaacgc cagcttcaag 240
   atgggcatca agttcaccaa ctggcgcacg cccggccgcg gcgaggcgac cgcgcgcccg 300
   cacggtgact cggtcgacca ctactaccac ctgttcggcc tgctgccgaa ccacgagaac 360
   cagccgctgt cgcactactg gacgcacaag aagctcagcg gtcagaccga cgagccgttc 420
   gactacgcgt gctaccgcga gcccccgatc atggaccaga agctgtcgcc ccggttcctg 480
   gacggcacca agtggaccaa ctacgcgtgg cacttcgacg cgaacctggt cgccgacttc 540
   ctccggcgct tcgccaccac gaagcagggc gcggtccaca tcgaggacaa gatgaccgag 600
   gcggtcatcg accagcgcgg ccacgtgtcc gcgctcagga ccgagcaggg ccgcacgatc 660
   gacggcgacc tgttcatcga ctgctccggg ttccgcggcc tgctgatcaa ccaggccatg 720
   aaggagccgt tcctggacat gagcgaccac ctgctcaacg acagcgcggt cgccacccag 780
   atcccgcacg acgacgacgc cgagggcgtc gagccctaca ccggctcgat cgcgatgtcg 840
   tcgggctggt cctggaagat cccgatgctg ggccgcttcg gcaccggcta cgtgttctcc 900
   tcccggttcc agacgcgcga cgacgccacg ctggagttct gcaacatgtg gggcgtcgac 960
   cccgagaccc agccgctcaa ccacatcaag ttccgggtcg gccgcaaccg gcgcacctgg 1020
   gtcaagaacg tggtctcgat cggcctggcg tcctgcttcc tggagccgct ggagtcgacc 1080
   ggcatctact tcgtctacgc ggcgctgtac cagctggcga agcacttccc ggacaagaac 1140
   ttcgactcga tcctgcagga ccgcttcaac gcggagatcg agacgatgtt cgacgactcc 1200
   cgggacttcg tgcagggtca cttcagcttc gccccgcgca acgacacggc gttctggcag 1260
   gcgtgcaagg agctgaagct ggcggacgac ttccaggaga aggtccgcat gtacaaggcg 1320
   ggcctggcgg tgaacatgcc gatcaccgac gagtcgacct actactccag cttcgacgcg 1380
   gagttccgca acttctggaa caactcgaac tactactgca tcttcgccgg gctggactac 1440
   ctgcccgacc acaccctgcc cgccctcgcg gcgcggccgg acgtgctgga ctccgccgac 1500
   gcggtgttcg cggagatcaa gcagaagcag aaggaactca tggcgacgct gcccaccacc 1560
   cacgagtacc tcaagcagct gcacggcaag tga 1593
<210> 14
   <211> 1593
   <212> DNA
   <213> Lechevalieria aerocolonigenes
<400> 14
   atggaagacc acaggatctc caatgtcgtc gttcttggtg gtggcaccgc aggctggatg 60
   accgccagtt atctgggtaa ggcgctcggc aggcatgcga cgatcaccgt gctggaggcg 120
   ccgtcgattc cgaagatcgg tgtcggtgag gccaccgtcc cgaatctgca caagacgttc 180
   ttcgacttct tgggtattcc cgaggaggag tggatgcggg agtgcaacgc gagtttcaag 240
   atggccgtga aattcgtcaa ctggcgcacg cccggtgaag gcgaggccgt cggcaggcaa 300
   ctgcgcggca ggcccgacca ctactaccac ctgttcggcc tgctgcccac gcaccagaac 360
   ctgccgctgt cgcactactg ggcggccaag agcctcggcg ggcagaccac cgagcagttc 420
   gactacgcgt gctaccgcga gtcgccgatc atcgacgccc gcctgtcgcc ccggcacgcc 480
   gacggtacca agtggacgaa ctacgcctgg cacttcgacg ctcatctggt cgcggacttc 540
   ctcaagcggt tcgccatcca gaagcagggc gtcgtgcacg tccaggacga gatgaccgac 600
   gcgacgatcg accagcgcgg ccacatcacg gccctcagga cgaaggaggg gcgcacgatc 660
   gagggcgacc tcttcgtcga ctgctccggc ttccgcgggt tgctgatcaa caagctgatg 720
   cgcgagccgt tcctcgacat gagcgaccat ctgctcaacg acagcgccgt cgccaccgcc 780
   gtgccgcacg acgacgaggc gcacggggtc gagcccgcca cgtcggcgat cgcgatgaag 840
   tccggctgga cgtggaagat cccgatgctc gacaggttcg gcaccggcta cgtctactcc 900
   agcaggttcg ccgactccga caccgccgcc agggacctgt gcgcgctgtg gaacctcgac 960
   cccgaggagc agccgctcaa ccacgtcaag ttccgcgtcg gccgcaaccg ccgcgcgtgg 1020
   gtgcgcaact gcgtcagcat cggcctgtcg tcgtgcttcc tggagccgct cgagtcgacc 1080
   ggcatctact tcatcacggc cgcgctgtac cagctgggca agcacttccc ggacaagcgg 1140
   ttcgacccgg tgctgatcga gtcgttcaac cgtgagatcg agacgatgtt cgacgactcg 1200
   cgggacttcc tgcaggcgca cttcacgctg gcgccgcgca cggacacgcc gttctggcgt 1260
   gcctgcaagg atctcgtgct gccggaggac atccagcaca agatcgcgca gtaccgggcg 1320
   ggactcgcga tcaacatgcc ggtcaccgac gaggccacct actacgagaa cttcgaggcc 1380
   gagttccgca acttctggac caacagcagc tactactgca tcttcgccgg cctggacgtg 1440
   ctgcccgacc acccgttgcc cacgctggct ttccggccgg aggcgatgga gggtgccgaa 1500
   ccggtgttcg cgcagatcaa gcagaagcag gccgagctgc tgaagacgct gccgaccgtc 1560
   cacgagtacc tgcgcgagtt gcacgggcgc tga 1593
<210> 15
   <211> 17
   <212> DNA
   <213> artificial
<220>
   <223> primer
<400> 15
   ggsgtsggsg argcsac 17
<210> 16
   <211> 20
   <212> DNA
   <213> artificial
<220>
   <223> primer
<400> 16
   gggatctycc asgtccascc 20
<210> 17
   <211> 28
   <212> DNA
   <213> artificial
<220>
   <223> primer
<400> 17 cccctgaagc ttggaggatg tcggtgtc 28
<210> 18
   <211> 28
   <212> DNA
   <213> artificial
<220>
   <223> primer
<400> 18
   caggtcccgg actagttcag cgggcatg 28
<210> 19
   <211> 30
   <212> DNA
   <213> artificial
<220>
   <223> primer
<400> 19
   ccgacaaagc ttggaggtcg cttatggctg 30
<210> 20
   <211> 30
   <212> DNA
   <213> artificial
<220>
   <223> primer
<400> 20
   cggcgactag ttcacttgct gtgcagatga 30
<210> 21
   <211> 31
   <212> DNA
   <213> artificial
<220>
   <223> primer
<400> 21
   aactccaagc ttggagggaa ttcgatatga c 31
<210> 22
   <211> 25
   <212> DNA
   <213> artificial
<220>
   <223> primer
<400> 22
gcggtgcgac tagtctactt gccgt 25
<210> 23
   <211> 30
   <212> DNA
   <213> artificial
<220>
   <223> primer
<400> 23
   acgcgaaagc ttggaggact tccgtggcac 30
<210> 24
   <211> 26
   <212> DNA
   <213> artificial
<220>
   <223> primer
<400> 24
   cgccgggcgg actagtctac gccgag 26
<210> 25
   <211> 31
   <212> DNA
   <213> artificial
<220>
   <223> primer
<400> 25
   ccgactaagc ttggagggct gtttcatgac c 31
<210> 26
   <211> 28
   <212> DNA
   <213> artificial
<220>
   <223> primer
<400> 26
   acgccgacta gtttacgcgc cgtgcagg 28
<210> 27
   <211> 31
   <212> DNA
   <213> artificial
<220>
   <223> primer
<400> 27
   gtgatcaagc ttggaggaca tttccacatg g 31
<210> 28
   <211> 26
   <212> DNA
   <213> artificial
<220>
   <223> primer
<400> 28
   gcccagccgg actagttcac ttgccg 26
<210> 29
   <211> 29
   <212> DNA
   <213> artificial
<220>
   <223> primer
<400> 29
   ggtgtaaagc ttggaggctg tccggatgg 29
<210> 30
   <211> 27
   <212> DNA
   <213> artificial
<220>
   <223> primer
<400> 30
   ggccacgcac tagttcagcg cccgtgc 27

## Description

### Field of the Invention

The invention relates to a novel halogenase, to methods for transferring halogen to a substrate, cells comprising the novel halogenase and uses of such halogenase and cells.

### Background of the invention and state of the art

Halogenated compounds are omnipresent in a variety of technological fields. They can be found as the active ingredients in pharmaceuticals, insecticides or herbicides. Lacquers, cosmetics, paints and plastics are produced through chemical synthesis with halogenated compounds as intermediates. But halogenated compounds are not only generated artificially, but also formed by living organisms. About 3800 halogenated natural products have been isolated until now (Gribble, 2003, The Handbook of Environmental Chemistry, Vol.3, Natural Production of Halogenated Compounds, Springer Verlag, Berlin). One particular rich source for halogenated metabolites are the actinomycetes, a subdivision of the gram positive bacteria. The halogenation reactions in these microorganisms are catalyzed by halogenases. Based on their mechanism of action halogenases can be divided into two large groups: haloperoxidases and perhydrolases form one group (van Pee et al., 1996, Annu. Rev. Microbiol, 50, 375-99) and the FADH₂-dependent halogenases constitute the other group (Kling et al. 2005, Biocombinatorial Approaches for Drug Finding, Enzymatic incorporation of halogen atoms into natural compounds, Ed. W. Wohlleben, T. Spellig, B. Müller-Tiemann, Ernst Schering Research Foundation Workshop 51, Springer Verlag, Berlin, 165-194). In contrast to haloperoxidases, is the latter type of halogenases able to catalyze the formation of carbon-halide bonds with high regioselectivity. For halogenating activity they require halide ions (chloride, bromide, iodide), molecular oxygen and FADH₂, which is produced from flavin adenine dinucleotide (FAD) and nicotine amide adenine dinucleotide (NADH) by a flavin reductase.

A number of halogenase genes are known from the reference P.E. Hammer et al., Appl. Environ. Microbiol. 63:2147-2154 (1997). A method for transferring a halogen to a substrate in a regiospecific manner is known from the reference WO 01/44447 A1.

Most halogenases are very specific for the substrate and thereby their applicability in in vivo or in vitro synthesis of halogenated compounds is rather limited. Accordingly, it is desirable to obtain halogenases, which are capable of transferring halogen to a comparatively broad range of substrates in order to allow a high number of different halogenation reactions with just one tool or a limited number of tools.

### Technical problem of the invention.

Accordingly, it is an object of the invention to provide a halogenase which is capable of transferring a halogen to a broad range of substrates. It is further an object of the invention to provide means and methods for transferring a halogen to a broad range of substrates using just one halogenase.

### Principles of the invention and preferred embodiments.

For solving the above mentioned technical problem the invention provides an isolated protein or enzyme comprising or consisting of a sequence according to Seq.-ID 1, which is effective to transfer a halogen to a carbon atom of an organic substrate.

These proteins or enzymes are halogenases, which are capable of transferring a halogen to a carbon atom of an organic compound as a substrate, wherein such halogen transfer is possible to a comparatively broad range of substrates. This means that the transfer may take place to substrates, which - although sharing a common structural feature - may vary considerably in structure. Furthermore, the transfer is regiospecifical, since these halogenases are FADH₂-dependent halogenases. Accordingly, the transfer occurs in the presence of an oxidant, a halogen donor, and FADH₂, or a FADH₂ source. The substrate is typically a molecular compound comprising of an indol-, or anthracene-group. Functional fragments of the said proteins or enzymes possess the same properties.

Only such proteins or peptides with said identity are comprised in the invention, which have the above-mentioned functionality being a FADH₂ dependent halogenase. The term functional fragments of proteins or enzymes according to the invention relates to partial sequences of sequence Seq.-ID 1 comprising a sequence of at least 10, preferably at least 20, more preferably at least 30, amino acids with above mentioned identity values, wherein only such fragments are functional, which exhibit preferably a regiospecific halogenation reaction in the assay described in detail in the Examples.

FADH₂-dependent halogenases need the presence of FADH₂, an oxidant, like molecular oxygen, and a halogen source, like halide ions, for transferring the halogen to the substrate. FADH₂ may be supplied in a variety of different manners. First, FADH₂ may be added to systems, which are not capable of producing endogenous FADH₂. Second, FADH₂ may be generated in vitro or in vivo by reducing FAD with NADH, NADPH or ferredoxin in the presence of a reductant. As a reductant a reductase capable of catalyzing an electron transfer from NADH, NADPH or ferredoxin to FAD is applicable. Examples are provided in the reference WO01/44447. Further, an organometallic cofactor regenerating system like pentamethylcyclopentadienyl-rhodium-bipyridine complex may be used as the reductant. As a source of halide ions generally any inorganic or organic compound capable of dissociating halide ions under the reaction conditions of the halogen transfer is suitable. Examples are alkaline halides, like Li-halides, Na-halides or K-halides, alkaline-earth halides, like Ca-halides and Mg-halides, and ammonium-halides. The term halides comprises fluorides, chlorides, bromides and iodides. Preferred sources of halide ions are LiCl, NaCl, KCI, CsCl, MgCl₂, CaCl₂, BaCl₂**,** NH₄Cl, and respective bromides.

The invention further relates to a method for transferring a halogen to a substrate comprising contacting the substrate with a halogenase of the invention or with a functional fragment thereof in the presence of an oxidant, a halogen donor, and FADH₂ or a FADH₂ source. The FADH₂ source may comprise FAD, NADH and a reductant, preferably a organometallic cofactor regenerating system, in particular a pentanethylcyclopentadienyl-rhodium-bipyridine complex.

The transfer may occur as an in vitro or in vivo reaction. In case of the in vivo reaction this is carried out in a cell comprising a heterologous nucleic acid sequence coding for a halogenase of the invention or a functional fragment thereof. The cell is cultured under usual culturing conditions applicable for the cell type under addition of the substrate, if not present endogenously, halide ion source, and components of the FADH₂ source, so far such components are not present endogenously. In case of the in vitro (cell-free synthesis) reaction the substrate, halide ion source, protein or enzyme of the invention, or functional fragment thereof, and FADH₂ (or the components of a FADH₂ source) are contacted with each other in an aqueous solution, typically a usual buffer, having a pH in the range from 4 to 10, preferably from 6.5 to 8.5, more preferably 7 to 8, and at temperatures in the range from 0°C to 65°C, preferably 15°C to 50°C, more preferably 20 to 40°C. In the in vitro variant, it may be favorable to immobilize the protein or enzyme of the invention e.g. by covalently binding directly to a matrix, by binding via immobilized antibodies against the protein or enzyme or against an antigen fused with the protein or enzyme, by binding of a first coupling compound, like biotin, being fused with the protein or enzyme, with a immobilized second coupling compound, like avidin, or by embedding into a polymer matrix, like methacrylate polymer.

For the purpose of in vivo synthesis, in particular in isolated host cells, the invention further relates to a transformed host cell expressing a heterologous protein or enzyme according to the invention. Examples for useable host cells are bacterial cells, fungal cells, microbial cells, and plant cells. Preferred cells are bacterial cells, in particular antibiotic producing bacterial cells, like the Actinomyces and Streptomyces species. Herein, the term heterologous relates to a protein, which is not naturally expressed in the host cell or not naturally expressed in the extend as in the transformed cell. Transformation of the host cell may be achieved by introducing a foreign nucleic acid according to the sequence Seq.-ID 8, which code for the sequence Seq.-ID 1 into the cell, wherein the foreign nucleic acid is controlled by an appropriate regulatory element. Introduction may be performed using usual vehicles like plasmids or cosmids. The foreign nucleic acid may be absent in the non-transformed cell or not present in the number of copies as present in the transformed cell. Accordingly, the invention also relates to the use of a host cell of the invention for producing a modified metabolite, wherein the modified metabolite differs from the naturally produced metabolite in that an indol- or anthracene-group of the metabolite is halogenated. The production is carried out by culturing the host cell of the invention for a defined period of time, as mentioned above. After culturing, the modified metabolite is either collected with the culture supernatant or by disrupture or lysis of the cells and subsequent collection of the obtained solution comprising the cell fluids. The modified metabolite is then in either case isolated from the obtained solution using well known techniques and finally purified to the desired purity degree.

In the following the invention is described in more detail by way of non-limiting examples.

### Example 1: Identification of novel halogenases

### Example 1.1: Primer design and PCR-based screening for novel halogenase genes

Sequence alignment studies of FADH₂-dependent halogenases, revealed regions of highly conserved amino acid sequences. Based on these findings a set of degenerated oligonucleotide primers were designed, taking into account the biased codon usage of actinomycetes. The two primers are as follows: 5'-GGSGTSGGSGARGCSAC-3' (abbreviated herein after as primer#1) and 5'-GGGATCTYCCASGTCCASCC-3' (abbreviated herein after as primer#2). These primers were used to detect homologous halogenase genes in other organisms through a PCR screening approach. The size of the expected amplified DNA fragment was estimated to be app. 700 bp.

A genetic screening for novel tryptophan-like halogenase genes with the above mentioned primer was conducted. The genomic DNA used as templates for the PCR reactions originated from a pool of actinomycetes strains proprietary to the inventor. Genomic DNA was isolated using the NucleoSpin^{®} kit from Macherey-Nagel according to the specification of the manufacturer. PCR was carried out with a PCR-cycler from MJ Research (PTC-100) under the following conditions. The initial denaturation temperature was 94°C for 3min, followed by 30 cycles of 94°C (60 s), 58°C (90 s) and 72°C (60 s). At the end the final extension temperature was 72°C for 5 min.

The PCR reaction mixture consisted of: 1 µl genomic DNA, 5 µl 10 x Buffer (Roche), 1 µl primer#1 (50 pmol), 1 µl primer#2 (50 pmol), 2 µl dNTPs (10 mM), 0.5 µl Taq Polymerase (Roche), 2.5 µl DMSO, 37 µl H₂O.

Out of this pool a total of 8 actinomycetes strains gave a positive PCR signal, indicating the presence of tryptophan like halogenases. Positive fragments with the expected size of approximately 700 bp were cloned into the pBluescript^{®} vector (Stratagene) using *E*. *coli* DH5α and *E. coli* XL1 Blue cells (Stratagene, Heidelberg, Germany) and sequenced. To obtain the full length sequences of the halogenase genes, genomic cosmid libraries from the eight positive tested strains were constructed in *E*. *coli* DH5α using pOJ436 as cosmid vector (Beye et al., 1998, GENOMICS 49, 317-320 and Burgtorf et al., 1998, GENOMICS 52, 230-232).

It follows a short description of the library construction and screening process. Extracts of homogenized bacterial culture suspensions were embedded in 0.5% "low melting point agarose" (SeaPlaque GTG, Biozym) and incubated with 2 mg/ml HEW-Lysozym (Roth) for 14 h at room temperature, followed by treatment with 1 mg/ml Proteinase K (Merck) for 24 h at 50°C. The embedded DNA was partially digested with S*au*3Al, extracted with Gelase (Epicentre) and dephosphorylated with methods that are known in the art. Ligation of the genomic DNA fragments into cosmid pOJ436 (Kieser et al., 2000, Practical Streptomyces Genetics, The John Innes Foundation, Norwich, England.) previously cut with 750 ng *Bam*Hl, desalting, in vitro λ-packaging (Gigapack III Gold Packaging Extract, Stratagene) and subsequent transduction of the ligation mixture into *E. coli* DH5α (Invitrogen) gave the desired cosmid libraries. Cosmid clones were randomly picked in 384 well plates and spotted onto nylon filters (Amersham). After a growth cycle, colonies were further processed as described by Nizetic et al., 1991, PNAS 88:3233-7. Cosmids harbouring the tryptophan-like halogenase genes were identified by southern hybridization with nonradioactive labelled DNA probes. Homologous DNA probes were generated by using the above described cloned PCR-fragments (in pBluescript^{®}) as templates and the PCR DIG Probe Synthesis Kit (Roche). The PCR reaction mix contained: 1 µl plasmid DNA containing an internal halogenase fragment, 5 µl 10 x buffer, 5 µl PCR DIG labeling mix, 1 µl of primer#1 (50 pmol) and 1 µl of primer#2 (50 pmol), 0,75 µl enzyme-mix and 36.25 µl H₂O. PCR was carried out using the same conditions as described earlier. Southern hybridization and all cloning and DNA modifications techniques used in this work are described in detail in Sambrook et al., 1989 (Molecular cloning: A laboratory manual, 2nd ed. Cold Spring Harbor Laboratory, Cold Spring Harbor, NY) and are known in the art. Inverse sequencing of the identified cosmids gave the complete halogenase gene sequences provided in the sequence listing Seq.-ID 8. The entire amino acid sequences are provided in the sequence listing Seq.-ID 1

The strains and the corresponding halogenases are listed in table 1.

**Table 1**

| **Strain** | **Halogenase** | **Seq ID No.** |
|---|---|---|
| *Streptomyces* Tü 1892 | Hal14 | 1 |

### Example 1.2: Cloning into a suitable expression vector, expression and cell disruption

The complete genes of the seven identified halogenases were each cloned into pUWL201oriT. This vector is a suitable expression vector for *S*. *lividans* TK23, equipped with a constitutive promoter *ermE* as well as thiostrepton resistance gene as selection marker. The primers used for subcloning the entire genes into pUWL201oriT are listed below:

| | |
|---|---|
| >Hal14 | |
| Primer-FW14: | 5'- CCCCTGAAGCTTGGAGGATGTCGGTGTC -3' |
| Primer-RV14: | 5'- CAGGTCCCGGACTAGTTCAGCGGGCATG -3' |

*S. lividans* TK23 was transformed with all seven constructs by protoplast transformation as specified by Kieser et al., 2000, Streptomyces Genetics, The John Innes Foundation, Norwich, England. Transformants were selected for thiostrepton (25 µg/ml) resistance and one recombinant clone from each transformation was selected for cultivation.

Pre-culture: *S. lividans* TK23 tansformants, harbouring the recombinant halogenases, were grown in 50 ml TSB-medium (Tryptic Soy Broth, Beckton Dickinson) supplemented with 25 µg/ml thiostrepton for 2 days at 30°C and 160 rpm in 2 baffled 100 ml flasks with one spring. The main cultures (100 ml TSB-medium, 500 ml flasks with 2 baffles and one spring) were inoculated with 1 to 5 ml of the pre-culture and grown for 2 days under the same conditions. Cells were harvested by centrifugation (5000 rpm, 20 min) and the resulting cell pellets were washed with potassium phosphate buffer (10 mM, pH 7.2) and resuspended in 2.5 ml of the same buffer containing 1 mg/ml lysozyme. The cells were broken up using a French press and after centrifugation (20,000 rpm, 30 min) the soluble fractions were used in the assays.

### Example 1.3: Assay condition for screening

The enzyme assays with a total volume of 200 µl contained 100 µl crude extract of a halogenase expressed in *S*. *lividans* TK23 strain, 2 µl crude extract of a flavin reductase expressed in *E*. *coli* JM109 (DE3) (pEE1001), 469 units catalase, 0.5 mM substrate, 10 µM FAD, 20 mM NaCl, 3 mM NADH in potassium phosphate buffer (10 mM, pH 7.2).

The crude extract of flavin reductase was prepared as described below: *E. coli* JM109 (DE3)(pEE1001) (Spyrou et al., 1991, J. Bacteriology, Vol 173, 12, 3673-3679) was grown overnight in 5 ml LB-medium supplemented with 100 µg/ml Ampicillin at 37°C and 180 rpm. This culture was used to inoculate the main culture (200 ml, LB-medium + 100 µg/ml Ampicillin, 37°C and 180 rpm). After 36 h the bacteria were pelleted by centrifugation and washed with 30 ml potassium phosphate buffer (10 mM, pH 7.2). Cells were resuspended in 2 ml of the same buffer and disrupted by sonification. Cell debris were removed by centrifugation and the resulting protein extract, containing the flavin reductase activity, was used in the assay.

The assay mix was incubated over night at 30°C with agitation (160 rpm). The reaction was terminated by heating to 95°C for 10 min and the resulting protein precipitate was removed by centrifugation. The supernatant was analyzed for the occurrence of halogenated compound with the below described analytical method.

### Example 1.4: HPLC Analytical method

Samples were analyzed with the below listed LC-UV-MS system and method parameters:
LC: Waters Alliance 2790 system with photodiode array detector
Column: GROM-SIL 120 ODS-4 HE 3µm (Length 40 mm, I.D. 4 mm)
Pre-column: Phenomenex C18 ODS Octadecyl (Length 4 mm, I.D. 3.0 mm) Mass spectrometer: Micromass QTOF2
Mode: ESI+; Capillary: 2.70kV, Cone: 33V; Source temp.: 120°C; N₂-
Desolvation flow: 600 l/h at 250°C, Injection volume: 15 µl
Binary gradient:
Solvent A: ddH₂O + 0.1% formic acid
Solvent B: acetonitrile

| time [min] | solvent A [%] | solvent B [%] | curve | flow [ml/min] |
|---|---|---|---|---|
| 0 | 98 | 2 | 1 | 1 |
| 5 | 98 | 2 | 6 | 1 |
| 9 | 20 | 80 | 6 | 1 |
| 13 | 20 | 80 | 6 | 1 |
| 14 | 98 | 2 | 6 | 1 |
| 16 | 98 | 2 | 6 | 1 |

The recorded data sets were screened for potential halogenated products by analysis of mass spectrometry and UV-data. Chlorine (³⁵Cl; 75.8%) and bromine (⁷⁹Br; 50.7%) have highly abundant isotopes (³⁷Cl ; 24.2%), (⁸¹Br; 49.3%), respectively, which gives these halogenated compounds a distinct isotopic distribution pattern. An enzymatic halogenation of the chemical compounds was considered to be successful if: i) bioconversion products displayed the required mass peak as well as the expected halogenated isotope pattern and ii) displayed a reasonable UV spectrum when compared to that of their respective educts and iii) could be verified in a repetitive, second conversion experiment in which the required mass, the isotopic pattern, the UV spectrum and the retention time were in agreement with the previously obtained data.

### Example 1.5: Results

The halogenases listed in table 1 were tested for their halogenating activity against different chemical substrates. All enzymes were cloned in pUWL201oriT and expressed in *S*. *lividans* TK23. To demonstrate the functional integrity of the cloned genes and the suitability of the selected expression system, L-tryptophan was chosen as a first substrate for the activity assay. With the exception of enzyme Hal908, all halogenases displayed a chlorinating activity against L-tryptophan, thus proving that the isolated gene sequences encode for functional enzymes and that these enzymes were successfully expressed. The seven halogenases of the present invention are all FADH₂ dependent halogenases. In addition to halide ions and molecular oxygen, they require FADH₂ for halogenating activity. In *vivo* the necessary FADH₂ is supplied by a flavin reductase, which produces FADH₂ from FAD and NADH. The same system was applied to the here described assay in which a flavin reductase expressed in *E*. *coli* JM109 (DE3), FAD and NADH were part of the assay-mix. However, the addition of exogenously supplied flavin reductase is not a mandatory requirement for halogenating activity. Experiments without the addition of flavin reductase also generate halogenated products. Studies conducted by Unversucht et al., 2005, Adv. Synth. Catal., 347, 1163-1167 have shown, that the direct contact between halogenase and reductase is not absolutely necessary for halogenating activity. The reductase simply serves as a FADH₂ supplier. It is therefore possible to substitute the flavin reductase with any other FADH₂ producing system e.g. with the organometallic cofactor regenerating system (pentamethylcyclopentadienyl)rhodium-bipyridine complex [Cp*Rh(bpy)(H₂O)]²⁺ or by adding FADH₂ itself.

Further investigations were aimed at exploring the substrate tolerance of the halogenases. Enzymes displaying relaxed substrate specificity should be able to cope with a structural more diverse set of substrates. The following compounds were used in the assays: L-trypotphan, D-trypotphan, 4-fluoro-DL-tryptophan, 4-methyl-DL-trypotphan, 5-bromo-DL-tryptophan, 5-fluoro-L-tryptophan, 5-fluoro-DL-tryptophan, 5-amino-DL-tryptophan, 5-hyrdoxy-DL-tryptophan, 5-methyl-DL-tryptophan, 5-methoxy-DL-tryptophan, 6-bromo-DL-tryptophan, 6-chloro-D-tryptophan, 6-fluor-DL-tryptophan, 6-methyl-DL-tryptophan, 7-methyl-DL-tryptophan, H-P-β-Benzothienyl)-D-Ala-OH, H-β-(3-Benzothienyl)-DL-Ala-OH, Melatonin, Serotonin, 1-aminoanthracen, indole-3-acetamide. The results are summarized in table 2. Only enzymatic conversions that met the criteria mentioned under 1.4 are listed in table 2. For the halogenase a list is provided, listing the different substrates (row 1), the degree of halogenation monohalogenation or dihalogenation (row 2) and the respective HPLC retention time. Retention times are in minutes and were acquired with the analytical system described in detail in Example 1

**Table 2: halogenation reactions**

| **Enzyme Hal14** | | |
|---|---|---|
| D-tryptophan | +1xCl | 7.11 |
| | | |
| L-tryptophan | +1xCl | 7.10 |
| | | |
| 4-fluaro-DL-tryptophan | +1xCl | 7.31 |
| | +1xCl | 8.06 |
| | | |
| | +1xCl | 7.46 |
| 4-methyl-DL-tryptophan | +1xCl | 8.15 |
| | | |
| 5-amino-DL-Tryptophan | +1xCl | 0.85 |
| | | |
| 15-bromo-DL-tryptophan | +1xCl | 7.55 |
| | | |
| 5-nuoro-DL-Tryptophan | +1xCl | 7.33 |
| | | |
| 5-fluoro-L-tryptophan | +1xCl | 7.28 |
| | | |
| 5-hydroxy-DL-Tryptophan | +1xCl | 5.85 |
| | +1xCl | 6.28 |
| | | |
| 5-methoxy-DL-tryptophan | +1xCl | 7.23 |
| | | |
| 5-methtyl-DL-Tryptophan | +1xCl | 7.46 |
| | | |
| 6-bromo-DL-tryptophan | | |
| | | |
| 6-fluoro-DL-tryptophen | | |
| | | |
| 6-chloro-D-tryptophan | | |
| | | |
| 6-methyl-DL-tryptophan | | |
| | | |
| (7-methyl-DL-tryptophan | +1xCl | 7.47 |
| | +1xCl | 8.25 |
| | | |
| 1-aminoanthracen | +1xCl | 9.57 |
| | +1xCl | 9.74 |
| | | |
| H-beta-(3benzothienyl)-D-Ala-OH | | |
| H-beta-(3benzothienyl)-DL-Ala-OH | | |
| | | |
| indole-3-acetamide | +1xCl | 7.84 |
| | | |
| melatonin | | |
| | | |
| serotonin | +1xCl | 4.49 |
| L-tryptophan | +1xBr | 7.30 |
| | +1xBr | 8.04 |
| | | |
| 5-bromo-DL-tryptophan | +1xBr | 7.57 |
| | | |
| 5-fluoro-DL-tlyptophan | | |
| | | |
| | +1xBr | 7.49 |
| 5-methtyl-DL-tryptophan | +1xBr | 8.24 |

The results clearly demonstrate that the identified halogenase, possesses a broad substrate and halogen donor tolerance. It is able to mono and dihalogenate different tryptophan, indol, and anthracene derivatives. In addition, it is able to utilize not only chloride, but also bromide as the halide donor. The halogenating activity is thereby regiospecific. Regiospecific means, that the formation of carbon-halogen bonds only takes place at specific locations in the substrate. The chosen compounds in this study are only a small selection of possible substrate and are standing representative for a much large group of structural diverse substrates.

### Example 2: in vitro large-scale preparation of halogenated products.

To obtain enough starting material of halogenated products, suitable for structural characterization, large scale preparation of some selected halogenase substrate combinations were carried out. Through the knowledge of the exact conformation of the halogenated products, conclusion about the regiospecificity of the involved halogenases can be made. The halogenases of the present invention are regiospecific.

### Example 2.1: Assay condition for large-scale preparation.

*S. lividans* TK23 and *E*. *coli* JM109 (DE3) harbouring recombinant plasmids were cultivated and processed as described under 1.2 and 1.3.

The enzyme assays with a total volume of 50 ml contained:
- 25 ml crude extract of a halogenase expressed in *S. lividans* TK23 strain,
- 500 µl crude extract of a flavin reductase expressed in *E. coli* JM1 09 (DE3),
- 117250 units catalase,
- 15 mg substrate,
- 10 µM FAD,
- 20 mM NaCl or 20 mM MgCl₂
- 3 mM NADH in potassium phosphate buffer (10 mM, pH 7.2)

The assay-solution was incubated at 30°C for 16 h with agitation (160 rpm). The use of the reductase/FAD-catalase system was aimed at improving the overall conversion rates of potential biocatalysis products. The omission of the flavin reductase gave qualitative the same product spectrum but the overall conversion rates were significant reduced when compared to conversion rates with reductase enzyme. In addition, the substitution of NaCl by MgCl₂ also had a beneficial effect on the observed conversion rates.

### Example 2.2: Purification schema

The reaction was stopped by heating the assay solution to 95°C for 10 min. The resulting protein precipitate was removed by centrifugation and the turbid solution was filtered through a Millipore ultrafiltration membrane (regenerated Cellulose, Filter Code: YM3, NMWL: 3000). This step removes macromolecules with a molecular weight of 3000 Da and higher, which would encumber the subsequent purification process by HPLC chromatography. The clear filtrate, containing the halogenated compound, was lyophilized and the resulting residue was redissolved in 1-2 ml acetonitrile. The final purification was achieved by semipreparative HPLC (Waters, 600 Controller, 996 Photodiode Array Detector, XTerra PrepMSC₁₈column, 10 µm, 19x300mm, flow: 10ml/min). A gradient from water (+0.05 % TFA) to acetonitrile was used, whereby the exact composition and time of the gradient varied from case to case.

### Example 2.3: Structure Elucidation

The halogenated derivatives were characterized using 1 D- and 2D-NMR-experiments (¹H. ¹³C, H,H-COSY (Long Range-Correlation Spectroscopy), HMQC (Heteronuclear Multiple Quantum Coherence), HMBC (Heteronuclear Multiple Bond Connectivity)) in combination with UV and ESI spectrometry data. All spectra were recorded on a Bruker Avance 600 (600 MHz) spectrometer, equipped with a 5 mm TXI high sensitive Cryoprobe head. Chemical shifts are reported in ppm relative to TMS and were adjusted to the solvent resonance, coupling constants (J) are reported in [Hz]. Abbreviation used throughout the tables: s = Singlet, d = Doublet, t = Triplett, q = Quartet, m = Multiplett, br = broad, ESI = Electrospray Ionization

The halogenase substrate combinations for which a large scale preparation was conducted are listed in table 3.

**Table 3**

| **Halogenase** | **Substrate** | **Halogenated Product** |
|---|---|---|
| Hal14 | 4-Methyl-Tryptophan | CBS000085 |
| Hal14 | 5-Methyl-Tryptophan | CBS000083 |

NMR, MS and UV-Data and the corresponding chemical structures are shown in the following.
**CBS000083**
Molecular Formula: C₁₂H₁₃N₂O₂Cl
UV/Vis (Maxima): 226 nm; 287 nm
ESI-MS:m/z= 236.1 [M+H-NH₃], 253.1 [M+H]

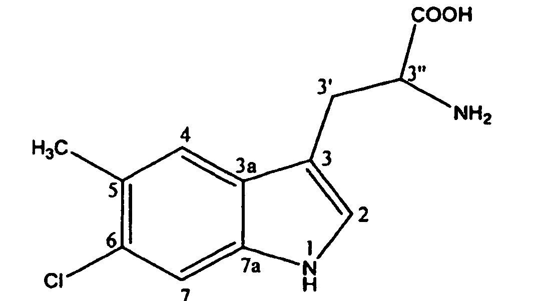

| | | |
|---|---|---|
| **¹H-NMR** (600 MHz, d₆-DMSO), **¹³C-NMR** (150.9 MHz, d₆-DMSO) | | |

| **Position** | **δ ¹H (ppm)** | **δ¹³C (ppm)** |
|---|---|---|
| N-1 | 10.96 s, 1H | - |
| C-2 | 7.20 d, 1.8 Hz, 1H | 125.6 |
| C-3 | - | 107.8 |
| C-3a | - | 126.4 |
| C-4 | 7.50 s, 1H | 119.8 |
| C-5 | - | 124.7 |
| C-5-CH₃ | 2.38 s, 3H | 20.1 |
| C-6 | - | 126.7 |
| C-7 | 7.40 s, I H | 111.2 |
| C-7a | - | 135.3 |
| C-3' | 3.07 dd, 7.9, 15.2 Hz, 1H 3.24 dd, 4.8, 15.2 Hz, 1H | 26.4 |
| C-3" | 3.79 br. s, 1H | 53.5 |
| COOH | not observed | 170.3 |
| NH₂ | not observed | - |

**CBS000085**
Molecular Formula: C₁₂H₁₃N₂0₂Cl
UV/Vis (Maxima): 230 nm; 283 nm
ESI-MS:m/z = 236. 1 [M+H-NH₃], 253.1 [M+H]

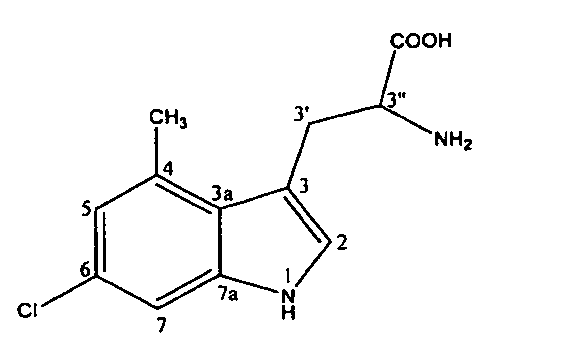

| | | |
|---|---|---|
| **¹H-NMR** (600 MHz, d₆-DMSO) | | |

| **Position** | **δ¹H (ppm)** | **δ¹³C (ppm)** |
|---|---|---|
| N-1 | 10.95 s, 1H | - |
| C-2 | 7.11 s, 1H | 124.8 |
| C-4-CH₃ | 2.61 s, 3H | 19.4 |
| C-5 | 6.75 br s, 1H | 120.0 |
| C-6 | - | - |
| C-7 | 7.19 d, 1.5 Hz, 1H | 108.9 |
| C-3' | 3.31^{a)}, 1H 3.02 m, 1H | 28.2 |
| C-3" | 4.40 br s, 1H | 53.4 |
| COOH | 8.22 d, 5.9 Hz, 1H | - |
| NH₂ | not observed | - |

| | | |
|---|---|---|
| ^{a)} overlapped by water signal ¹³C-signals were only detected indirectly through HMQC crosspeaks | | |

### Example 3: Sequences

In the following the sequences of the halogenases are provided. The assignments of the Seq.-ID nos are as following (enzyme/amino acid sequence/nucleotide sequence): Hal14/1/8,

### AMINO ACID SEQUENCE LISTINGS

Halogenase Hal14, SEQ ID No:1

### NUCLEIC ACID SEQUENCE LISTINGS

Halogenase Hal14, SEQ ID No:8

## Claims

1. Isolated protein or enzyme comprising or consisting of a sequence according to Seq.-ID 1which is effective to transfer a halogen to a carbon atom of an organic substrate.

2. Isolated protein or enzyme according to claim 1, wherein the transfer is regiospecifically.

3. Isolated protein or enzyme according to one of the claims 1 or 2, wherein the transfer occurs in the presence of an oxidant, a halogen donor, and FADH₂ or a FADH₂ source.

4. Isolated protein or enzyme according to one of the claims 1 to 3, wherein the substrate is a molecular compound comprising an indol-, indoline- or anthracene-group or respective hetero-atom substituted group.

5. Isolated nucleic acid coding for a protein or enzyme according to one of the claims 1 to 4 or a partial sequence thereof coding for a functional fragment of a protein or enzyme according to one of the claims 1 to 4.

6. Method for transferring a halogen to a substrate comprising contacting the substrate with a halogenase according to one of the claims 1 to 4, or a functional fragment thereof, in the presence of an oxidant, a halogen donor, and FADH₂ or a FADH₂ source.

7. Method according to claim 6, wherein the FADH₂ source comprises FAD, NADH and a reductant, preferably an organometallic cofactor regenerating system, in particular a pentanethylcyclopentadienyl-rhodium-bipyridine complex.

8. Method according to one of the claims 6 or 7, wherein the transfer occurs as an in vitro or in vivo reaction.

9. Method according to one of the claims 6 to 8, wherein the in vivo reaction is carried out in a preferably isolated cell comprising a heterologous nucleic acid sequence coding for a halogenase according to one of the claims 1 to 5, or a functional fragment thereof, in particular comprising a heterologous nucleic acid according to claim 6.

10. A transformed host cell expressing a heterologous protein or enzyme according to one of the claims 1 to 4.

11. The cell of claim 10, comprising a heterologous nucleic acid according to claim 5 under control of a regulatory element.

12. Use of a protein or enzyme according to one of the claims 1 to 4 for producing a molecule comprising a halogenated indol-, indoline or anthracene-group or respective hetero-atom substituted group.

13. Use of a of a host cell according to claim 10 or 11 for producing a modified metabolite, wherein the modified metabolite differs from the naturally produced metabolite in that an indol-, indoline or anthracene-group or hetero-atom substituted indol-, indoline or anthracene group of the metabolite is halogenated.

## Patentansprüche

1. Isoliertes Protein oder Enzym enthaltend oder bestehend aus einer Sequenz nach Seq.-ID 1, welche zum Übertragen eines Halogens an ein Kohlenstoffatom eines organischen Substrats wirksam ist.

2. Isoliertes Protein oder Enzym nach Anspruch 1, wobei das Übertragen regiospezifisch erfolgt.

3. Isoliertes Protein oder Enzym nach einem der Ansprüche 1 oder 2, wobei das Übertragen in Anwesenheit eines Oxidationsmittels, eines Halogendonors und FADH₂ oder einer FADH₂-Quelle erfolgt.

4. Isoliertes Protein oder Enzym nach einem der Ansprüche 1 bis 3, wobei das Substrat eine molekulare Verbindung enthaltend eine Indol-, Indolin-oder Anthracen-Gruppe oder eine entsprechende Heteroatom-substituierte Gruppe ist.

5. Isolierte Nukleinsäure codierend für ein Protein oder Enzym nach einem der Ansprüche 1 bis 4 oder eine Teilsequenz davon codierend für ein funktionelles Fragment eines Proteins oder Enzyms nach einem der Ansprüche 1 bis 4.

6. Verfahren zum Übertragen eines Halogens an ein Substrat, bestehend aus: Kontaktieren des Substrats mit einer Halogenase nach einem der Ansprüche 1 bis 4 oder einem funktionellen Fragment davon, in Anwesenheit eines Oxidationsmittels, eines Halogendonors und FADH₂ oder einer FADH₂-Quelle.

7. Verfahren nach Anspruch 6, wobei die FADH₂-Quelle FAD, NADH und ein Reduktionsmittel, vorzugsweise ein organometallisches Cofactor-Regenerationssystem, insbesondere einen Pentanethylcyclopentadienyl-Rhodium-Bipyridin-Komplex aufweist.

8. Verfahren nach einem der Ansprüche 6 oder 7, wobei das Übertragen als eine in vitro- oder in vivo-Reaktion erfolgt.

9. Verfahren nach einem der Ansprüche 6 bis 8, wobei die in vivo-Reaktion in einer vorzugsweise isolierten Zelle enthaltend eine heterologe Nukleinsäure-Sequenz codierend für eine Halogenase nach einem der Ansprüche 1 bis 5, oder ein funktionelles Fragment davon, insbesondere enthaltend eine heterologe Nukleinsäure nach Anspruch 6, ausgeführt wird.

10. Transformierte Wirtszelle, die ein heterologes Protein oder Enzym nach einem der Ansprüche 1 bis 4 exprimiert.

11. Zelle nach Anspruch 10, enthaltend eine heterologe Nukleinsäure nach Anspruch 5 unter Kontrolle eines regulatorischen Elements.

12. Verwendung eines Proteins oder Enzyms nach einem der Ansprüche 1 bis 4 zur Herstellung eines Moleküls enthaltend eine halogenierte Indol-, Indolin- oder Anthracen-Gruppe oder eine entsprechende Heteroatom-substituierte Gruppe.

13. Verwendung einer Wirtszelle nach Anspruch 10 oder 11 zur Herstellung eines modifizierten Metabolits, wobei der modifizierte Metabolit sich vom natürlich hergestellten Metabolit **dadurch** unterscheidet, dass eine Indol-, Indolin- oder Anthracen-Gruppe oder eine Heteroatom-substituierte Indol-, Indolin- oder Anthracen-Gruppe des Metabolits halogeniert ist.

## Revendications

1. Protéine ou enzyme isolée comprenant ou consistant en une séquence selon Séq.-ID 1 qui est efficace à transférer un halogène à un atome de carbone d'un substrat organique.

2. Protéine ou enzyme isolée selon la revendication 1, dans laquelle le transfert est régiospécifique.

3. Protéine ou enzyme isolée selon une des revendications 1 ou 2, dans laquelle le transfert a lieu en présence d'un oxydant, d'un donneur d'halogène, et de FADH₂ ou d'une source de FADH₂.

4. Protéine ou enzyme isolée selon une des revendications 1 à 3, dans laquelle le substrat est un composé moléculaire comprenant un groupe indole, indoline ou anthracène ou un groupe respectif substitué par des hétéroatomes.

5. Acide nucléique isolé codant pour une protéine ou enzyme selon une des revendications 1 à 4 ou une séquence partielle de celles-ci codant pour un fragment fonctionnel d'une protéine ou enzyme selon une des revendications 1 à 4.

6. Procédé pour transférer un halogène à un substrat comprenant: contacter le substrat avec une halogénase selon une des revendications 1 à 4, ou un fragment fonctionnel de celle-ci, en présence d'un oxydant, d'un donneur d'halogène, et de FADH₂ ou d'une source de FADH₂.

7. Procédé selon la revendication 6, dans laquelle la source de FADH₂ comprend FAD, NADH et un agent réducteur, de préférence un système de régénération de cofacteur organométallique, en particulier un complexe pentane-éthylcyclopentadiénylrhodium-bipyridine.

8. Procédé selon une des revendications 6 ou 7, dans laquelle le transfert a lieu sous la forme d'une réaction in vitro ou in vivo.

9. Procédé selon une des revendications 6 à 8, dans laquelle la réaction in vivo est exécutée dans une cellule de préférence isolée comprenant une séquence d'acide nucléique hétérologue codant pour une halogénase selon une des revendications 1 à 5, ou un fragment fonctionnel de celle-ci, en particulier comprenant un acide nucléique hétérologue selon la revendication 6.

10. Une cellule hôte transformée exprimant une protéine ou enzyme hétérologue selon une des revendications 1 à 4.

11. Cellule selon la revendication 10, comprenant un acide nucléique hétérologue selon la revendication 5 sous le contrôle d'un élément régulateur.

12. Utilisation d'une protéine ou enzyme selon une des revendications 1 à 4 pour produire une molécule comprenant un groupe indole, indoline ou anthracène halogéné ou un groupe respectif substitué par des hétéroatomes.

13. Utilisation d'une cellule hôte selon la revendication 10 ou 11 pour produire un métabolite modifié, dans laquelle le métabolite modifié diffère du métabolite naturellement produit en ce qu'un groupe indole, indoline ou anthracène ou un groupe indole, indoline ou anthracène substitué par des hétéroatomes est halogéné.
